(19) Europäisches Patentamt
European Patent Office
Office européen des brevets

(11) Numéro de publication : **0 410 998 B1**

(12) # FASCICULE DE BREVET EUROPEEN

(45) Date de publication du fascicule du brevet : **21.12.94 Bulletin 94/51**

(51) Int. Cl.$^5$ : **G01N 33/543,** G01N 33/52

(21) Numéro de dépôt : **89904571.0**

(22) Date de dépôt : **06.04.89**

(86) Numéro de dépôt international : **PCT/FR89/00156**

(87) Numéro de publication internationale : **WO 89/10564 02.11.89 Gazette 89/26**

(54) **DISPOSITIF ET PROCEDE POUR LA DETERMINATION QUALITATIVE ET QUANTITATIVE RAPIDE DE LA PRESENCE D'UN LIGAND REACTIF DANS UN FLUIDE.**

(30) Priorité : **28.04.88 FR 8805668**

(43) Date de publication de la demande : **06.02.91 Bulletin 91/06**

(45) Mention de la délivrance du brevet : **21.12.94 Bulletin 94/51**

(84) Etats contractants désignés : **AT BE CH DE FR GB IT LI LU NL SE**

(56) Documents cités :
**EP-A- 0 021 261**
**EP-A- 0 133 700**
**EP-A- 0 173 375**
**EP-A- 0 249 851**
**EP-A- 0 279 097**
**FR-A- 2 514 511**
**US-A- 4 459 358**

(73) Titulaire : **CIS BIO INTERNATIONAL**
**RN 306**
**F-91000 Saclay (FR)**
Titulaire : **QUICK-TEST**
**94, rue Edouard-Vaillart**
**F-92300 Levallois-Perret (FR)**

(72) Inventeur : **MARCHAND, Joseph**
**No14 Le Colombier**
**Rue de Marcoule**
**F-30130 Pont-Saint-Esprit (FR)**
Inventeur : **TOLEDANO, Jacques**
**55, rue des Orteaux**
**F-75020 Paris (FR)**

(74) Mandataire : **Ores, Irène et al**
**CABINET ORES**
**6, Avenue de Messine**
**F-75008 Paris (FR)**

**EP 0 410 998 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention est relative à un dispositif et à un procédé pour la détermination qualitative et quantitative rapide d'un ligand réactif présent dans un fluide. L'invention s'applique plus particulièrement , mais non limitativement, à la détection de la présence d'anticorps ou d'antigènes dans un fluide biologique, à la détection de substances toxiques, de virus ou de bactéries dans un fluide quelconque (eaux, produits fluides des industries agro-alimentaires, effluents industriels, fluides biologiques, etc,...), au dosage chimique de substances (telles qu'hormones, vitamines, enzymes, médicaments, etc...) présentes dans un fluide (notamment biologique).

Les méthodes de détermination de la présence ou de la concentration de substances antigéniques dans des fluides biologiques, par voie immunologique, sont à présent bien connues ; elles sont basées sur la formation d'un complexe entre la substance antigénique à déterminer et un ou des anticorps, l'un des composants du complexe pouvant être marqué par un élément radioactif (tel que $^{125}I$, par exemple) pour permettre sa détection et/ou son analyse quantitative après séparation de l'antigène ou de l'anticorps marqué complexé, de l'antigène ou de l'anticorps marqué non complexé.

Dans les méthodes de détermination immunologique par compétition, la substance antigénique contenue dans l'échantillon de liquide à analyser, entre en compétition avec une quantité connue d'antigène marqué pour une quantité limitée de sites de fixation de l'anticorps : la quantité d'antigène marqué liée à l'anticorps est inversement proportionnelle à la quantité d'antigène contenue dans l'échantillon.

Les tests immunométriques utilisent un anticorps marqué : la quantité d'anticorps marqué associée au complexe est proportionnelle à la quantité de substance antigénique contenue dans l'échantillon.

Des tests immunométriques particulièrement adaptés a la détection d'antigènes polyvalents, c'est-à-dire de substances antigéniques capables de se complexer avec au moins deux anticorps en même temps, consistent à utiliser une quantité d'anticorps non marqué lié à un support solide insoluble dans le liquide à analyser, et une quantité d'anticorps soluble portant un indicateur, tel qu'un isotope radioactif, qui permet la détection ou la détermination quantitative du complexe ternaire formé entre l'anticorps en phase solide, l'antigène et l'anticorps marqué. Ces méthodes consistent à mettre tout d'abord en contact l'anticorps lié à la phase solide avec l'échantillon à analyser, pour extraire l'antigène de l'échantillon, par formation d'un complexe binaire entre l'anticorps en phase solide et l'antigène. Après une période d'incubation, le support solide est lavé pour en éliminer le résidu d'échantillon liquide, y compris l'antigène éventuel n'ayant pas réagi, puis mis en contact avec une solution contenant une quantité connue d'anticorps marqué. Après une seconde période d'incubation pour permettre à l'anticorps marqué de se complexer avec l'antigène lié au support solide par l'intermédiaire de l'anticorps non marqué, le support solide est lavé une seconde fois pour enlever l'anticorps marqué n'ayant pas réagi, puis la présence d'anticorps marqué sur le support solide lavé est détectée (par exemple par mesure du rayonnement émis, si l'indicateur est un élément radioactif) et éventuellement soumise à un processus de détermination quantitative.

Ces méthodes sont connues sous le nom de méthodes "sandwich" ou "bi-site", du fait que l'antigène porte deux anticorps liés à sa surface en deux sites différents. Elles sont décrites par WIDE dans "Radioimmuno assay Methods" (Kirkham et Hunter, E. et S. Livingstone, Ed., Edimbourg, 1970, p. 199-206). Des applications spécifiques (test de détection de l'antigène lié à l'hépatite) sont décrites dans le Brevet des Etats-Unis d'Amérique N 3 867 517 ; des variantes de ces méthodes ("simultanées" ou "inverses") sont décrites par JEONG et al. dans "Comparison of RIA with a single-incubation two-site immunoradiometric assay (IRMA) as applied to the determination of human thyroïd stimulating hormone (HTSH)", Bio-Rad Laboratories, 1979 ; dans le Brevet des Etats-Unis d'Amérique N 4 174 384 (marquage de deux anticorps par un chromophore fluorescent (fluorescéine) et par un chromophore qui absorbe la lumière émise par la fluorescéine) ; dans le Brevet des Etats-Unis d'Amérique N° 4 098 876. Ces méthodes utilisaient initialement des anticorps polyclonaux, dont la sensibilité est insuffisante en raison de l'existence d'une réactivité croisée avec d'autres antigènes. Le Brevet Français HYBRITECH N° 81 15030 publié sous le N° 2 487 983, a proposé de remplacer dans ces tests immunométriques, les anticorps polyclonaux par des anticorps monoclonaux, pour augmenter de façon importante la sensibilité de ces méthodes.

Le Brevet Français LIOTTA N° 82 16973, publié sous le N° 2 514 511, décrit un dispositif et un procédé pour déterminer la présence d'antigènes dans des fluides biologiques, par la méthode ELISA, tout en éliminant les opérations de dilution et de lavage qu'elle nécessite et en réduisant la durée de la période d'incubation. Le dispositif proposé comprend une matrice formée d'un ruban en papier de nitrocellulose ou de diazobenzyloxyméthyle (DBM), en gel poreux tel que polyacrylamide, agarose ou collagène, dans les pores duquel l'antigène peut être piégé ou bien il peut être réticulé avec le gel par l'intermédiaire des groupes amine du ligand et des groupes carboxyle portés par la matrice, ou bien d'un ruban de cellulose ou de fibres de matière plastique à l'intérieur duquel sont piégées des particules ou des billes qui contiennent le ligand. Conformément à

ce brevet LIOTTA, le dispositif comprend : - une première zone qui contient des antigènes et des anticorps liés à une enzyme, ces anticorps étant situés dans ladite première zone de telle manière qu'ils soient eliminés de cette première zone lorsqu'ils ont réagi avec des antigènes (non liés) contenus dans l'échantillon à tester, traversant cette première zone, mais qu'ils n'en soient pas éliminés en l'absence de tels antigènes, - et une seconde zone contenant une substance capable de réagir avec lesdits anticorps liés à une enzyme, pour produire une réaction colorée révélant la présence desdits anticorps. De façon spécifique,le dispositif de dosage immunologique décrit dans le Brevet LIOTTA comprend un stratifié à trois couches de matrice poreuses dont la première est imprégnée d'un anticorps spécifique lié à une enzyme, la seconde couche poreuse contient un antigène de référence immobilisé (lié), et la troisième couche contient un substrat produisant une couleur, qui réagit avec l'enzyme liée à l'anticorps. L'antigène libre à doser présent dans l'échantillon à analyser, mis en contact avec la première couche, diffuse dans la seconde,puis dans la troisième couche. L'antigène libre présent dans l'échantillon entre en compétition avec l'antigène de référence lié, immobilisé dans la seconde couche, pour se combiner à l'anticorps lié à une enzyme. Si l'anticorps lié à une enzyme se combine à l'antigène libre, il diffuse librement dans la troisième couche et produit une réaction colorée. Par contre, si l'échantillon à analyser ne contient pas d'antigène, tous les anticorps liés à une enzyme possèdent des sites de liaison libres et se combinent avec l'antigène immobilisé présent dans la seconde couche ; l'anticorps lié à une enzyme, qui se combine à l'antigène immobilisé dans la seconde couche, ne diffuse pas dans la troisième couche et aucune réaction colorée ne se produit. Le Brevet Français LIOTTA 87 08007, publié sous le N° 2 599 845 décrit une variante de ce dispositif, qui ne comprend que deux couches, dont la couche supérieure porte l'antigène avec les deux anticorps réactifs et dont la couche inférieure contient un substrat chromogène, les deux zones, la zone de piégeage et la zone de substrat, pouvant également être juxtaposées. Les couches poreuses à travers lesquelles diffuse l'antigène de l'échantillon, combiné à l'anticorps lié à une enzyme, jouent donc le rôle d'un filtre sélectif qui ne laisse passer que l'antigène combiné à l'anticorps marqué par une enzyme, mais ne fonctionne pas si l'anticorps n'est pas combiné à de l'antigène, c'est-à-dire en l'absence d'antigène.

Le Brevet américain HYBRITECH N° 4 632 901 (et la Demande Internationale PCT correspondante N° 85/05451) décrit un procédé et un dispositif de réalisations d'immunoessais qui ne nécessitent pas le recours à des étapes d'incubation de longue durée associées à plusieurs lavages , et permettent, par leur simplicité, leur mise en oeuvre par le médecin, à sa consultation, et même par le patient chez lui. Le dispositif en question comprend un premier élément qui est poreux et qui est constitué par une membrane ou un filtre auquel est lié un anticorps, monoclonal ou polyclonal, qui reconnaît l'antigène que l'on cherche à identifier, et un deuxième élément, qui est absorbant et comporte des passages capillaires perpendiculaires à ses surfaces supérieure et inférieure ; ce second élément est en communication capillaire avec la membrane, ou analogue, poreuse, qui forme le premier élément du dispositif, et présente une dimension de pores telle qu'il induise la traversée de liquide à travers le premier élément sans application de moyens externes. La membrane poreuse filtrante peut être du Nylon portant des groupes $NH_2$ auxquels les anticorps sont liés par covalence, par couplage à l'aide de glutaraldéhyde et l'élément absorbant peut être en matière filtrante fibreuse telle que fibres d'acétate de cellulose, de polyester, de polyoléfine, etc... Ces deux éléments peuvent être séparés l'un de l'autre par un autre élément poreux (en polyéthylène par exemple) qui ne fixe pas d'anticorps de façon non- spécifique et empêche que de l'anticorps marqué ne se fixe de façon non-spécifique sur la surface supérieure de l'élément absorbant.

De même, la Demande de Brevet Européen ABBOTT LABORATORIES N° 186 100 décrit un dispositif qui vise à déterminer la présence ou la quantité d'une substance dans un échantillon à tester, et qui comprend une matrice fibreuse poreuse, non-absorbante, telle que du verre, imprégnée d'un polymère hydrophobe qui forme un revêtement de surface sur au moins une partie de la matrice et auquel est fixé un réactif, par exemple un anticorps ou un antigène, capable de réagir avec la substance recherchée dans l'échantillon, ce dispositif pouvant être associé à une couche de matière absorbante sous-jacente et/ou à une couche de matière fibreuse telle que fibres de verre ou papier-filtre en cellulose, qui recouvre la matrice fibreuse et peut jouer le rôle de "pré-filtre".

La Demande de Brevet Européen MUREX CORPORATION N° 206 561 fait également connaître un dispositif de diagnostic qui comprend un élément filtrant comportant au moins une zone de réaction associée à au moins une zone périphérique, un élément absorbant associé uniquement à la zone périphérique du premier élément et un élément de maintien du filtre en position appropriée. La "réaction" qui se produit dans la zone de réaction peut aussi bien être une séparation par filtration qu'un couplage immunologique et c'est dans la même zone qu'apparaissent les signaux de lecture de la réaction. L'échantillon liquide à tester parvient sur le filtre par un entonnoir dont l'orifice de décharge est calculé de telle manière que son diamètre soit suffisant, en combinaison avec la pression du liquide dans l'entonnoir , pour que le liquide se décharge sur la surface supérieure du filtre mais ne traverse pas ce dernier, la pression hydrostatique étant, par suite, ajustée de telle manière que le liquide pénètre dans le filtre par gravité et est diffusé à travers ce dernier par action capillaire,

sans le traverser perpendiculairement de part en part.

Tous ces dispositifs ont en commun la présence d'un élément filtrant, poreux, à travers lequel s'écoule l'échantillon liquide à tester, des moyens étant prévus, le cas échéant, pour ralentir l'écoulement du liquide. Toutefois, un tel élément filtrant ne permet pas un temps de contact suffisant entre le réactif lié à l'élément filtrant, et la substance recherchée dans l'échantillon liquide, et ne permet pas, de ce fait, l'obtention d'une réaction dont la sensibilité soit telle qu'elle soit apte à détecter de très faibles concentrations de ladite substance dans l'échantillon ; de plus, l'écoulement de l'échantillon contenant, le cas échéant, un complexe ligand-récepteur, tel que, notamment, un complexe antigène-anticorps, à travers un élément filtrant est susceptible d'engendrer des erreurs de lecture.

La présente invention s'est en conséquence donné pour but de pourvoir à un dispositif et à un procédé pour la détermination qualitative et quantitative rapide de la présence d'un ligand réactif, dans un fluide, qui répondent mieux aux nécessités de la pratique que les dispositifs et procédés visant au même but proposés dans l'Art antérieur, notamment en ce que le dispositif conforme à l'invention présente une sensibilité très supérieure à celles indiquées dans l'Art antérieur, puisqu'il peut détecter de très faibles concentrations de ligand, notamment d'antigène, dans un fluide, notamment biologique, qui peuvent être aussi basses que 5 picogrammes, alors que les procédés de l'Art antérieur ne permettent pas de détecter des concentrations aussi faibles, et ne sont sensibles qu'à partir de quelques μg ; en ce qu'il permet de réaliser deux réactions entre un réactif et une substance à détecter, en deux endroits totalement séparés, ce qui donne lieu à des réactions très spécifiques et évite ainsi à coup sûr les réactions croisées et les faux positifs ou négatifs, sans recourir aux méthodes par compétition ; en ce qu'il est capable de mettre en oeuvre plusieurs types d'anticorps monoclonaux simultanément et s'applique à tous les modes de lecture des résultats, tels que colorimétrie, fluorescence, chimioluminescence, radioactivité, etc... et en ce qu'il permet de réaliser des dosages quantitatifs.

La présente invention a pour objet un dispositif pour la détermination qualitative et quantitative rapide de la présence d'un ligand réactif, dans un fluide, du type comprenant une zone de réaction contenant un réactif marqué, notamment un anticorps ou un antigène marqué, destiné à réagir avec le ligand recherché, et une zone de révélation, lequel dispositif est caractérisé en ce qu'il comprend, en combinaison :

- une première zone de réaction qui comprend une membrane au moins temporairement imperméable laquelle zone est, prévue pour recevoir un échantillon de fluide à tester et laquelle membrane est destinée à être associée à au moins un réactif convenablement marqué, apte à reconnaître le ou les ligands recherché(s) éventuellement contenu(s) dans ledit échantillon, et à des moyens de perméabilisation de ladite membrane ;
- une deuxième zone de réaction, distincte de la première et au moins temporairement isolée de celle-ci par la membrane susdite, comprenant une phase solide contenant un réactif de référence, non marqué, apte à reconnaître le ou les ligands que l'on cherche à identifier et au moins temporairement close du côté opposé à celui où se trouve la première membrane, par une deuxième membrane au moins temporairement imperméable, et
- une troisième zone de réaction au moins temporairement isolée de la deuxième zone de réaction susdite par ladite deuxième membrane, qui contient des moyens de révélation de la réaction qui s'est éventuellement produite dans la première ou la deuxième zone de réaction, ledit dispositif étant en outre caractérisé en ce qu'au moins l'une des deux membranes susdites est réalisée en un matériau imperméable pendant une première période de temps suffisante pour permettre à la réaction qui doit se produire dans la zone de réaction intéressée, d'avoir lieu, ledit matériau étant tel qu'il est perméabilisé au bout de cette première période de temps, pour laisser passer des produits de la réaction qui y a eu lieu, vers la zone de réaction suivante, les moyens de perméabilisation de la ou des membranes étant constitués par une substance de dissolution ou un agent de lyse d'une ou des membrane(s).

Selon un mode de réalisation du dispositif conforme à la présente invention, les moyens de perméabilisation d'une ou des membrane(s) sont indépendants de la membrane concernée.

Selon un autre mode de réalisation avantageux du dispositif conforme à la présente invention, les moyens de perméabilisation d'une ou des membrane(s) sont incorporés à la membrane concernée.

Selon une disposition avantageuse de l'invention, les matériaux au moins temporairement imperméables utilisés pour réaliser une ou les membrane(s) susdite(s), sont pris dans le groupe qui comprend, notamment, des hydrates de carbone, des protéines notamment de la gélatine, des lipides, des matières plastiques.

Selon un autre mode de réalisation avantageux du dispositif conforme à la présente invention, la première zone de réaction est propre à recevoir une phase solide, avantageusement supportée par la première membrane, qui comprend au moins un réactif convenablement marqué, éventuellement associé à un agent de lyse de ladite membrane.

Conformément à l'invention, le réactif marqué est marqué de manière appropriée, notamment par une enzyme, par un radioisotope, par un produit chimique chromophore fluorescent ou chimioluminescent et il peut

être avantageusement un anticorps ou un antigène.

Egalement conformément à l'invention, le matériau dont est constituée au moins la première membrane est tel que celle-ci est dissoute au bout d'un temps approprié, par la phase liquide aqueuse de l'échantillon à tester.

Conformément à la présente invention, l'agent de perméabilisation est constitué par un agent de lyse, tel qu'une enzyme appropriée, de la membrane concernée, qui peut être soit incorporé dans celle-ci, soit associé à ladite membrane par introduction dans le dispositif conforme à l'invention, soit associé dans la phase solide qui comprend le réactif susdit.

Selon encore un autre mode de réalisation avantageux du dispositif conforme à la présente invention, la phase solide contenant un réactif de référence, non marqué, qui se trouve dans la deuxième zone de réaction, est constituée par un support insoluble sur lequel est fixé ledit réactif (anticorps ou antigène, notamment) non marqué, qui correspond au ligand que l'on cherche à détecter, lequel support insoluble est choisi dans le groupe qui comprend notamment les microsphères, les microplaques ou autres.

Selon un mode de réalisation avantageux du dispositif conforme à la présente invention, la troisième zone de réaction contient un substrat chromogène lorsqu'il est prévu que la lecture de la réaction doit se faire par colorimètrie.

Selon un autre mode de réalisation avantageux du dispositif conforme à la présente invention, le dispositif comporte entre la première et la deuxième zones de réaction, une zone intermédiaire qui comprend un réactif marqué, auquel cas la première zone de réaction comprend un réactif non marqué.

Selon une disposition avantageuse de ce mode de réalisation, la zone intermédiaire comprend au moins une couche de structures vésiculaires multicouches dans lesquelles est incorporé au moins un réactif propre à être libéré au bout d'un temps approprié par un agent de lyse approprié, tel, notamment, qu'une enzyme.

Selon une autre disposition avantageuse de ce mode de réalisation, la zone intermédiaire est formée d'une membrane intermédiaire intercalée entre la première membrane susdite et la deuxième zone de réaction, laquelle membrane intermédiaire est avantageusement associée à un réactif marqué, auquel cas la première membrane est associée à un réactif de même nature que la membrane intermédiaire susdite.

Le mode de réalisation du dispositif conforme à l'invention, qui comporte trois zones de réaction, permet d'effectuer des déterminations qualitatives de la présence ou de l'absence de ligands réactifs dans un fluide, notamment d'antigènes ou d'anticorps dans un fluide biologique.

Le mode de réalisation du dispositif conforme à l'invention qui comporte une zone supplémentaire permet de procéder à des dosages semi-quantitatifs de tels ligands.

Conformément à la présente invention, il est également possible de réaliser des dosages quantitatifs, en associant la troisième zone du dispositif à un appareillage de quantification connu en lui-même, tel que RIA, spectrophotomètre, etc.

Selon un mode de réalisation avantageux du dispositif conforme à la présente invention, les zones de réaction susdites sont superposées pour former un étage supérieur contenant la première zone de réaction, un étage médian contenant la deuxième zone de réaction et un étage inférieur contenant la troisième zone de réaction, et éventuellement un étage intermédiaire entre l'étage supérieur et l'étage médian, qui contient la zone de réaction intermédiaire.

La présente invention a également pour objet un procédé de détermination qualitative et quantitative rapide de la présence d'un ligand réactif dans un fluide, qui est caractérisé en ce qu'au cours d'une première étape, un échantillon du fluide à analyser est mis en contact avec un réactif marqué, tel qu'un anticorps ou un antigène marqué, notamment, dans une zone temporairement isolée des zones dans lesquelles ont lieu les étapes ultérieures du procédé, pendant un temps suffisant pour permettre à la réaction ligand-réactif de se produire, au bout duquel l'isolement de la zone dans laquelle a eu lieu ladite réaction, est rompu à l'aide d'une substance de dissolution ou d'un agent de lyse approprié présent dans ladite zone, transporté simultanément audit fluide vers une membrane temporairement imperméable qui est dissoute au contact de ladite substance ou agent, -laquelle dissolution ou lyse progressive et programmée de la membrane d'isolement est simultanée à ladite réaction-, pour permettre au produit éventuel de la réaction d'entrer en contact, au cours d'une deuxième étape, avec un second réactif, non marqué, correspondant au ligand que l'on cherche à identifier dans l'échantillon de fluide susdit, dans une seconde zone temporairement isolée de la zone qui la suit, après quoi l'isolement de la seconde zone est rompu de manière progressive, programmée et simultanée à l'aide de ladite substance de dissolution ou dudit agent de lyse pour permettre au produit de la première ou de la deuxième réaction d'entrer en contact, au cours de la troisième étape du procédé, avec des moyens de révélation de la présence - ou de l'absence - du ligand que l'on cherche à détecter.

Selon un mode de mise en oeuvre du procédé conforme à la présente invention, l'échantillon de fluide est mis en contact, au cours de la première étape du procédé, avec un réactif non marqué et la seconde étape du procédé est précédée d'une étape intermédiaire au cours de laquelle le complexe ligand-réactif non marqué

formé au cours de la première étape, est mis en contact avec un réactif marqué de même nature que le réactif mis en oeuvre dans la première étape, dans une zone temporairement isolée de l'étape suivante.

Selon une modalité avantageuse de ce mode de mise en oeuvre, le réactif non marqué mis en contact avec l'échantillon de fluide à analyser au cours de la première étape du procédé, est introduit à raison d'une quantité déterminée qui correspond à une quantité connue de ligand tel que celui que l'on cherche à détecter, le ligand en excès, c'est-à-dire non lié au réactif au cours de la première étape, est piégé, au cours de l'étape intermédiaire du procédé, par le réactif marqué, sur lequel ledit excès de ligand se fixe, d'où il résulte que la reaction de révélation de la présence éventuelle du ligand dans le fluide à analyser n'est positive que si le taux de ligand dans ledit fluide est supérieur à la quantité susdite, permettant ainsi une détermination semi-quantitative du ligand présent dans ledit fluide.

Selon un mode de mise en oeuvre préféré du procédé conforme à la présente invention, la première étape du procédé est une phase d'absorption et la deuxième étape du procédé est une phase de désorption, et la troisième étape du procédé est une phase d'absorption.

Lorsque le procédé conforme à l'invention comporte une étape intermédiaire intercalée entre la première et la deuxième étape du procédé, cette étape intermédiaire est, comme la première étape, une phase d'absorption.

Outre les dispositions qui précèdent, l'invention comporte encore d'autres dispositions, qui ressortiront de la description qui va suivre.

L'invention sera mieux comprise à l'aide du complément de description qui va suivre, qui se réfère aux dessins annexés dans lesquels :

- la Figure 1 est une représentation schématique en coupe orthogonale, d'un mode de réalisation du dispositif conforme à la présente invention ;
- la Figure 2 représente un schéma des étapes du procédé de détermination qualitative de la présence d'antigènes ;
- la Figure 3 représente un schéma des étapes du procédé de détermination qualitative de la présence d'anticorps, et
- la Figure 4 représente la courbe de dosage quantitatif de l'ACE présente dans du sérum humain.

Il doit être bien entendu, toutefois, que ces dessins et les parties descriptives correspondantes, sont donnés uniquement à titre d'illustration de l'objet de l'invention, dont ils ne constituent en aucune manière une limitation.

Le dispositif conforme à la présente invention, représenté à la Figure 1, comprend, de bas en haut, un support 7 qui porte une pastille de substrat chromogène 6, au-dessus de laquelle est disposée une membrane 3′ qui sera décrite de façon plus détaillée plus loin. Au-dessus de cette membrane 3′ est disposée une couche de microsphères 5 qui seront décrites de façon plus détaillée dans la suite du présent Mémoire descriptif. Les microsphères sont avantageusement, mais non nécessairement, protégées de part et d'autre par des couches de protection qui peuvent être en tous matériaux appropriés, et notamment en textile. Une seconde membrane, 3, est disposée au-dessus de la couche 5 de microsphères ; la structure 2 forme conjointement avec le support 7 et un cadre intermédiaire 8, le bâti du dispositif. Dans l'échancrure 9 en forme de coupe de la structure 2 est insérée une pastille 1 de préférence lyophilisée, d'un réactif qui reconnaît le ligand dont la détection sera réalisée à l'aide du dispositif conforme à la présente invention.

Les ligands que l'on cherche à détecter à l'aide de ce dispositif sont des antigènes et des anticorps, mais aussi toutes sortes de substances chimiques ou biologiques. Les applications du procédé et du dispositif conformes à la présente invention sont très nombreuses ; l'on peut citer parmi elles : le diagnostic des affections virales et microbiennes, les dosages enzymatiques, le dépistage des maladies auto-immunes et de la maladie d'Alzheimer, le dépistage précoce de cancers, la réalisation de groupages sanguins rapides, le dosage de médicaments circulants, la détection d'allergènes et le diagnostic d'allergies, le dosage des récepteurs membranaires, les dosages d'hormones, les dosages d'éléments chimiques, et notamment des anions et des cations de l'ionogramme. Bien entendu, cette énumération n'est pas exhaustive, ni limitative.

Les réactifs qui reconnaissent le ligand à détecter sont essentiellement des anticorps ou des antigènes, qui sont mis en oeuvre de préférence à l'état lyophilisé, sous forme de pastilles 1.

Les anticorps ou antigènes contenus dans la pastille 1 sont des anticorps ou antigènes marqués par un marqueur approprié tel qu'un radio-isotope, une enzyme, une substance fluorescente, un produit chimique susceptible de donner avec un substrat approprié une réaction colorée, etc.

La membrane 3 qui forme l'étage supérieur du dispositif est réalisée en matériau qui est temporairement imperméable, pendant une durée suffisante pour permettre à une goutte d'échantillon de fluide à examiner mise en contact avec la pastille 1, de réagir pour former, si le fluide contient effectivement le ligand à détecter, un complexe ligand-anticorps ou antigène marqué, la membrane 3 perdant son imperméabilité au bout de ce temps pour laisser passer en totalité le fluide contenant éventuellement le complexe susdit, jusqu'à l'étage médian.

Il est avantageux de réaliser la membrane 3 en un matériau qui est imperméable dans des conditions normales, mais est dissous en l'espace de 20 secondes à 10 minutes par une enzyme appropriée. Dans le cas où la membrane 3 est en gélatine, l'enzyme de lyse mise en oeuvre est avantageusement la collagénase. Il est avantageux d'inclure l'enzyme de lyse de la gélatine dans la pastille 1 d'anticorps ou d'antigène, car la durée nécessaire pour que le ligand et le réactif (anticorps ou antigène) contenu dans la pastille 1 réagissent entre eux, est du même ordre que la durée nécessaire à l'enzyme de lyse pour dissoudre la membrane 3. Il peut être avantageux de renforcer la membrane 3 de gélatine, en y noyant une structure en fibres de verre qui constitue le squelette de la membrane 3 après la dissolution de la gélatine. On trouvera plus loin d'autres exemples de membranes susceptibles d'être mise en oeuvre dans le cadre de la présente invention.

L'étage médian du dispositif conforme à la présente invention comprend une couche 5 de microsphères réalisées en tous matériaux appropriés tels que métaux et leurs alliages, polyacrylamide, etc., de diamètres variables compris entre 10 et 250 µm, portant un réactif (antigène ou anticorps), identique ou similaire au ligand que l'on cherche à détecter, destiné à retenir les réactifs marqués restés libres à l'étage supérieur.

Dans ces conditions, si l'on recherche un antigène dans une goutte de fluide biologique, en cas de réaction positive l'antigène présent dans ledit fluide se lie à l'anticorps contenu dans la pastille 1, sur la membrane 3, qui est dissoute par une enzyme appropriée dès que la réaction ci-dessus a eu lieu ; le fluide contenant le complexe antigène-anticorps précité traverse la couche de microsphères, 5, sans être retenu par ces dernières, puis parvient sur la membrane 3' qu'il dissout par l'intermédiaire de l' enzyme précitée contenue dans ledit fluide, pour permettre au fluide de parvenir dans la zone de révélation qui contient un substrat 8 qui est coloré par contact avec le fluide examiné.

En cas de réaction négative, c'est-à-dire si le fluide examiné ne contient pas d'antigène, l'anticorps marqué de l'étage supérieur est retenu par l'antigène non marqué fixé sur les microsphères et ne colore donc pas le substrat.

Les modalités sont les mêmes, mutatis mutandis, si c'est un anticorps que l'on recherche dans le fluide.

Si on veut obtenir une détermination semi-quantitative, le réactif de l'étage supérieur est non marqué, tandis qu'un étage intermédiaire fait suite à l'étage supérieur et comprend des structures vésiculaires multicouches ou des microcapsules à l'intérieur desquelles sont incorporés des réactifs marqués. Le réactif associé à la membrane 3, tel qu'anticorps par exemple, est introduit à raison d'une quantité qui correspond à une quantité déterminée d'antigène, soit x ng/ml d'antigène, par exemple 10 ng/ml d'antigène. Si le fluide testé contient une quantité plus élevée d'antigène, l'excès est fixé sur les anticorps marqués, incorporés aux structures vésiculaires de l'étage intermédiaire, dès l'ouverture de celles-ci sous l'action de l'enzyme de lyse, en sorte que l'on obtient des résultats par rapport à un seuil, c'est-à-dire des résultats semi-quantitatifs.

La Figure 2 illustre le dosage qualitatif d'antigènes, avec obtention d'une réaction positive : on y voit le complexe anticorps-antigène formé, désigné par la référence 10, traverser l'étage médian sans être retenu sur les microsphères, pour former une réaction positive. En effet, la totalité des anticorps marqués de la pastille 1 est saturée par les antigènes présents dans le sérum, au bout de 30 secondes environ, en sorte que lorsque le complexe saturé parvient, après la lyse de la membrane supérieure 3, après ces 30 secondes, sur les microsphères de la couche 5 sur laquelle sont fixés des antigènes de même nature que ceux qui sont recherchés dans le sérum, les microsphères ne peuvent fixer ces complexes saturés, qui, à travers la membrane 3' lysée, parviennent dans la zone de révélation.

De façon plus spécifique, le dosage illustré à la Figure 2 s'applique au dosage qualitatif de la présence d'antigènes de la syphilis.

Les anticorps marqués qui constituent le réactif de l'étage supérieur 13 sont des IgG humaines (500 picogrammes) provenant de sérums de contrôle positifs (Code 76751 Pasteur), marquées à la péroxydase (Merck) sur de la concanavaline A (méth. de GUESDON et AVRAMEAS, 1980). La pastille qui contient le réactif contient également, l'enzyme de lyse de la membrane, qui si cette dernière est en gélatine, est de la collagénase, auquel cas ladite pastille contient de préférence 5 unités de collagénase (collagénase Clostridium de Calbiochem). La membrane 3 peut également être une membrane en polycarbonate DMF, de 10µm d'épaisseur, comportant des pores de 3 µm bouchés à chaud par une solution éthanolique à 3 % de gélatine, la gélatine qui bouche les pores étant lysée par la collagénase au bout de 30 secondes. La membrane 3 est supportée par une structure en fibres de verre. Les anticorps polyclonaux marqués 14 utilisés comme réactifs, mis en contact avec les antigènes sériques 15 contenus dans la goutte de sérum mise en contact avec la pastille qui contient le réactif 14, forment un complexe anticorps marqué-antigène 10. Comme un sang positif en antigènes VDRL en contient environ 200 picogrammes par goutte de 50 microlitres, les 500 picogrammes d'anticorps marqués introduits à l'étage supérieur 13 sont suffisants pour capter les antigènes sériques 15 ; cet excès d'anticorps a d'ailleurs une incidence sur l'accélération de la vitesse de la réaction Ag-Ac. En effet, sur les 500 picogrammes d'anticorps marqués 14 de l'étage supérieur 13, 200 picogrammes vont se lier aux antigènes

sériques 15 pour former le complexe Ag-Ac 10, qui ne se fixera pas à la phase solide constituée par les microsphères 5 de l'étage médian 11. Ces microsphères (5 µl) en "Magnogel" (IBF) comportent un coating d'antigène VDRL-LATEX (1 ng/5 µl) sur protéine A + glutaraldéhyde (Réactif IBF) et VDRL, (Diag Pasteur Code 52675). Dès que la gélatine de la membrane 3 est lysée, 300 picogrammes d'anticorps marqués restés libres vont se fixer sur les antigènes VDRL-LATEX portés par les microbilles 5 de l'étage médian 11, tandis que les 200 picogrammes d'anticorps marqués saturés par les antigènes sériques, 10, vont traverser la membrane 3′ inférieure (identique à la membrane 3), dès que la gélatine qui, conformément à l'exemple présentement décrit, bouche ses pores est lysée, pour aboutir à l'étage inférieur 16, qui est la zone de révélation qui contient le substrat (S) avantageusement constitué par de la 3,3′,5,5′-tétraméthylbenzidine stabilisée (ICN-LAB. MILES) sur un support approprié tel que papier Whatman N° 1, par exemple ; par combinaison avec le substrat (S), le complexe 10 donne une réaction colorée (12) ; la coloration peut apparaître avec seulement 20 picogrammes si outre le 3,3′,5,5′-TMB, le substrat contient le complexe Glucose-Oxydase/B-D-Glucose qui produit de l'eau oxygénée, au contact de sérum, permettant ainsi l'amplification de la coloration.

La Figure 3 illustre le dosage qualitatif d'anticorps avec obtention d'une réaction positive : après 30 secondes environ, la totalité des antigènes marqués est saturée par les anticorps sériques, puis la membrane supérieure 3 est lysée, permettant le passage de tous les complexes anticorps-antigènes marqués, vers les microsphères de l'étage médian, auxquelles sont fixés des anticorps non marqués de même nature que les anticorps recherchés. Comme les épitopes (sites antigéniques) sont saturés par les anticorps sériques, ils ne sont pas reconnus par les anticorps fixés sur les microsphères et parviennent dans la zone de révélation, dans laquelle ils réagissent avec le substrat pour donner une réaction colorée.

L'exemple illustré à la Figure 3 s'applique plus particulièrement à la détection d'anticorps de la toxoplasmose.

L'étage supérieur 13 reçoit en tant que réactif constitué par des antigènes marqués, des antigènes de la toxoplasmose (Code 52721 Diag. Pasteur) avantageusement lyophilisés (250 picogrammes), marqués à la péroxydase (MERCK). La pastille lyophilisée contenant les Ag 17 contient une enzyme de lyse, qui dans le cas où la membrane est telle que décrite plus haut en relation avec la figure 2, est spécifiquement de la collagénase (5 unités) (Collagénase Clostridium de Calbiochem). Les antigènes marqués 17, utilisés comme réactif, mis en contact avec les anticorps sériques (polyclonaux) 18 contenus dans la goutte de sérum mise en contact avec la pastille qui contient le réactif 17, forment un complexe Antigène marqué-anticorps sérique 19. Comme un sang positif en anticorps anti-toxoplasmose en contient 100 à 150 picogrammes, les 250 picogrammes d'Antigènes marqués 17 saturent ces anticorps sériques 18 et les empêcheront de se fixer sur les microbilles (5µl) 20 - telles que microbilles de "Magnogel" (IBF) comportant un coating d'anticorps anti-toxoplasmose (1 ng/5 ùl) sur protéine A + Glutaraldéhyde (réactifs IBF ; anticorps Institut Pasteur) - qui se trouvent à l'étage médian 11. Dès la perméabilisation de la membrane 3, par lyse de la gélatine qui bouche les pores de la membrane de polycarbonate, comme décrit plus haut à titre d'exemple non limitatif, les antigènes marqués 17 non saturés par les anticorps sériques 18, se fixent sur les anticorps supportés par les microbilles 20, tandis que les complexes antigènes-marqués - anticorps, 21, franchissent la membrane inférieure 3′ dès qu'elle est à son tour perméabilisée, pour se combiner au substrat (S) qui peut être le même que décrit plus haut en relation avec la figure 2, en donnant une coloration, qui peut éventuellement être amplifiée comme également décrit en relation avec la Figure 2.

Les descriptions du dispositif conforme à la présente invention qui précèdent se sont référées aux modes de réalisation représentés aux figures 1 à 3 et ont donné comme exemples de membranes à lyse programmée essentiellement des membranes constituées par ou comprenant de la gélatine. Il est cependant clair que les membranes à lyse programmée peuvent, conformément à la présente invention, être réalisées en d'autres matériaux. Les exemples non limitatifs qui vont suivre ont pour objet d'une part, de décrire d'autres membranes à lyse programmée utilisables dans le cadre de la présente invention et d'autre part de décrire l'application du dispositif et du procédé de dosage conformes à la présente invention, au dosage d'antigènes présents dans du sérum ou des urines.

Exemple 1. Préparation de membranes en lipides

Les membranes en lipides peuvent être préparées à partir de nombreuses variétés de lipides amphiphiliques ou à partir de mélanges de lipides.

Les lipides qui conviennent le mieux à la formation de membranes imperméables sont les phospholipides associés ou non à du cholesterol, qui sont les constituants de base des membranes cellulaires.

Les phospholipides naturels (non synthétiques) ont en commun un même radical R -mais se différencient par leur groupement terminal :

| | |
|---|---|
| R - H | Acide phosphatidique |
| R - $CH_2$ - $CH_2$ - N - $(CH_3)_3$ | Phosphatidylcholine |
| R - $CH_2$ - $CH_2$ - $NH_3$ | Phosphatidyléthanol -amine |
| R - $CH_2$ - CH - $NH_3$<br>│<br>COO | Phosphatidylserine |
| R - $CH_2$ - CHOH - $CH_2$ - OH | Phosphatidylglycérol |
| R - $CH_2$ - CHOH - $CH_2$ - O - R' | Cardiolipine |
| R - $R_1$ complexe | Sphyngomyeline |

Il existe des phospholipides synthétiques offrant de plus grandes qualités de résistance, de conservation, etc.. Ce sont :

DMPC Dimyristoyl- Phosphatidylcholine
DPPC Dipalmitoyl-Phosphatidylcholine
DSPC Distearoyl-Phosphatidylcholine

1. Préparation de la solution lipidique :
   - Phospholipide 10 µmoles
   - Benzène 1 ml

   On agite au Vortex 1 minute.
2. On chauffe au bain-marie 40°C quelques minutes.
3. On dépose 12 µl sur un des supports suivants de diamètre 13 mm ; Nylon, Nitrocellulose, Papier de cellulose, Polyester, de porosité d'au moins 0,45 µ et d'épaisseur 100 µ.
4. Evaporation sous vide poussé ( - 60 mm Hg) du benzène et stockage immédiat sous vide ou dans de l'argon.
5. La lyse de ce type de membrane est réalisée à l'aide d'un détergent approprié et/ou de péroxydase.

   Test de lyse :

   On dépose sur une pastille 10 µl d'eau : la goutte reste immobile pendant au moins 1 heure.

   On dépose sur une pastille 10 µl de Tween 20 ou de Triton X-100 à 0,02 % et/ou de Péroxydase à 1 ng/µl :

   La goutte de 10 µl reste immobile pendant 45 secondes avant de traverser brusquement la membrane de support.
6. Etalonnage des membranes : on peut changer le délai de lyse en faisant varier la concentration de phospholipide ou la concentration de détergent-péroxydase, en procédant selon l'une des modalités décrites ci-après :

   6.1 Pour une membrane réalisée à partir d'une solution de 15 µmoles de phospholipide dans 1 ml de benzène : la goutte de Triton X-100 0,02 % ne passe qu'au bout de 90 secondes.

   6.2 Si l'on utilise du Triton X-100 à 0,01 % pour une même concentration de phospholipide, la lyse interviendra au bout de 5 minutes.

   6.3 Dans le cas d'une membrane préparée à partir d'une solution de 5 µmoles de phospholipide dans 1 ml de benzène : le Triton X-100 à 0,02 % traverse la membrane en 15 secondes, tandis qu'à 0,01 % le délai est de 50 secondes.

Remarque importante : Les détergents non-ioniques, tout en assurant une bonne protection de certaines solutions (ex : les anticorps marqués à la péroxydase) s'associent à cette même péroxydase pour lyser la membrane.

Exemple 2. Péparation de membranes en copolymères d'acrylates

1. On prépare la solution-mère suivante :
   - Propanol-2 (iso-propanol) 30 %
   - Copolymère acrylique/acrylate 40 %
   - OH-Propyl-Méthyl Céllulose 20 %
   - acetyl-trietyl Citrate 5 %
   - Chlorhydrate d'aluminium 5 %

On chauffe 10 minutes à 40°C au bain-marie.
On agite 1 minute 1 minute au Vortex.
On stocke hermétiquement fermé.

2. Avant de déposer sur un des supports décrits à l'exemple précédent, on dilue cette solution-mère dans de l'éthanol pur à raison de :
   1 volumes de solution acrylique mère
   5 volumes d'éthanol pur.
3. On dépose 10 µl sur le support de 13 mm de diamètre.
4. On sèche sous vide. On stocke sous vide en présence d'un dessicant.
5. Ce type de membrane est lysé par du PBS à pH 7,2.
   Test de lyse :
   On dépose une goutte de 10 µl de tampon PBS pH 7 : la traversée de la membrane est lente (environ 1 minute).
   On dépose 10 µl de PBS pH 7,2 : la traversée du support se produit au bout de quelques secondes.
6. Il est possible de raccourcir ou d'allonger le délai de lyse du film acrylique en faisant varier :
   - la composition de la solution-mère, (par exemple : plus ou moins de propanol-2),
   - la proportion d'éthanol : par exemple : à 9 volumes d'éthanol sur 10, le film est très vite perméable. A 4 volumes sur 5, le passage est plus lent.
7. Il est possible de faire varier le pH de lyse : en faisant varier la concentration de l'acétyl-triethyl citrate.

Exemple 3. Préparation de membranes en protéines

Les membranes en gélatine auxquelles il a été fait référence en relation avec la description du dispositif représenté aux figures 1 à 3, sont avantageusement préparées comme suit :

On prépare une solution-mère de composition suivante :
- Gélatine végétale 5 mg
- Eau déminéralisée 95 ml

On chauffe sous agitation magnétique à 50°C.
On dépose 10 µl sur un support tel que ceux décrits à l'Exemple 1.
On sèche sous vide poussé.
On stocke sous vide, en présence d'un dessicant.
La gélatine végétale est lysée par la collagénase.
Test de lyse :
On dépose 10 µl d'eau sur la pastille ainsi préparée. L'eau reste immobile plus d'une heure.
On dépose 10 µl d'une solution de collagénase à 1 UI/µl : la goutte traverse le support en 45 secondes.

Il est possible de retarder le passage de fluides à travers cette membrane protéique en incluant dans la solution qui la traverse de la collagénase ou toute autre enzyme protéolytique. La collagénase est choisie ici du fait qu'elle est compatible avec les réactions biologiques espérées au-dessus ou au-dessous de la membrane alors que d'autres enzymes protéolytiques sont toxiques, notamment pour les anticorps ou les protéines de marquage.

Il est également possible de faire varier le délai de traversée de cette membrane en variant les concentrations de gélatine ou les concentrations de collagénase. C'est ainsi qu'on réduit le délai avec moins de gélatine ou plus de collagénase et qu'on l'allonge avec plus de gélatine ou moins de collagénase.

Exemple 4. Préparation de membranes en hydrates de carbone

On prépare la solution-mère suivante :
D - Glucose 10 grammes

On chauffe 5 ml d'Eau déminéralisée à 70°C ; sous agitation au Vortex, on verse par petites quantités 10 g de D-Glucose, en agitant entre deux additions de D-Glucose. On obtient une solution épaisse à consistance de sirop.

On dilue 1 volume de ce sirop dans 1 volume d'éthanol pur 99°, puis on dépose 10 µl de cette solution sur l'un des supports décrits à l'Exemple 1 ; on sèche sous vide à 37°C. On stocke en présence d'un dessicant. Ce type de membrane est lysé en présence d'eau.

Test de lyse :
On dépose 10 µl d'eau sur :
1 - Le même support sans D-Glucose : traversée immédiate,
2 - Le support préparé avec le D-Glucose : traversée retardée de 35 secondes.

Le D-Glucose convient particulièrement brut car il permet d'obtenir du péroxyde au contact de la glucose oxydase, amplifiant ainsi le signal dans les dosages utilisant la péroxydase comme marqueur.

Exemple 5. Détection d'ACE présente dans du sérum

La Figure 4 représente une courbe de dosage de l'ACE contenue dans des échantillons de sérum humain.

Les échantillons testés ont un volume de 50 µlitres. 10 échantillons de même volume de sérum humain à tester et un échantillon de sérum humain test ne contenant pas d'ACE sont traités comme suit : on verse un échantillon sur la pastille lyophilisée 1 qui est supportée par la membrane 3 et qui contient 1250 picogrammes d'IgG1 murine anti-épitope 328.C de l'ACE marquée à la péroxydase.

Au bout de 30 secondes, la totalité des anticorps marqués a été saturée par les antigènes sériques. La lyse de la membrane 3, qui est, par exemple, en gélatine (renforcée par un réseau de fibres de verre) par la collagénase n'a lieu qu'après ces 30 secondes, ce qui provoque l'écoulement du sérum contenant les anticorps marqués saturés par les antigènes sériques, dans la deuxième zone de réaction, dans laquelle ils sont mis en contact avec les microbilles de la couche 5, revêtues des mêmes antigènes. Ils ne sont pas retenus par les antigènes qui revêtent les microbilles 5, car les sites Fab des anticorps marqués sont saturés par les antigènes sériques ; l'enzyme présente dans le fluide qui traverse la seconde zone de réaction, lyse alors la deuxième membrane 3′, ce qui provoque l'écoulement du fluide dans la troisième zone de réaction ; dans cette zone, la totalité des anticorps marqués liés aux antigènes sériques, arrive au contact de la pastille de substrat , qui est, par exemple, de la 3,3′,5,5′-tétraméthylbenzidine, et ils provoquent une coloration quasi-immédiate en présence de péroxydase. Une bandelette disposée sous le dispositif conforme à l'invention recueille le produit de filtration et révèle ainsi le signal-réponse qui indique le taux de matériel marqué qui traverse les microbilles

Les différentes concentrations en ACE de l'échantillon analysé, qui sont de 0,5 - 1 - 1,5 - 2 - 2,5 - 5 - 10 - 15 - 20 nanogrammes d'ACE /ml de sérum, soit 25-50-75-100-125-250-500-750-1000 picogrammes d'ACE dans les 50 µl d'échantillon à doser, sont représentées dans la Figure 4.

En raison des seuils de détectabilité très bas que permettent le dispositif et le procédé conformes à la présente invention, il est possible de réaliser des déterminations dans le cadre de la présente invention avec une seule goutte de sang de 50 µl.

Il est également possible de réaliser une pluralité de diagnostics conjointement en utilisant des microsphères portant différents marqueurs de diagnostic.

La sensibilité des dosages est de l'ordre de 5 à 125 picogrammes, selon la sensibilité de l'anticorps monoclonal mis en oeuvre : c'est pourquoi il est recommandé d'utiliser des anticorps monoclonaux ultraspécifiques.

Comme on l'a dit plus haut, les étages successifs du dispositif conforme à la présente invention constituent des phases présentant des affinités différentes vis-à-vis de l'eau ; c'est ainsi que l'étage supérieur est une phase d'absorption, l'étage médian est une phase de désorption et l'étage inférieur est une phase d'absorption. Cette alternance est obtenue, dans l'exemple décrit, grâce à l'établissement d'un gradient osmotique glucosé. Il en résulte que tous les liquides sont aspirés vers le substrat de l'étage inférieur, alors que, en cas de réaction négative, seul le matériel marqué est retenu sur les microsphères.

Exemple 6. Test qualitatif de détection d'anticorps anti-HIV sur sang total

6.1 Dispositif pour la détection d'anticorps anti-HIV sur sang total

| Matériau | Substances contenues | Quantité |
|---|---|---|
| Support en papier D.122 traité | Antigènes HIV marqués à la Glucose Oxydase (GP 40 et GP 120) | 50 ng |
| 1ère Membrane à lyse programmée (MLP) | lipides (cf exemple 1) | |
| Phase solide Nylon prèparé | Anticorps identiques à ceux recherchés | Excès puis lavages et blocking |
| 2ème MLP | lipides | |
| Nylon | TMB | 3 µl |
| | sur tampon Citrate 1M | 10 µl à pH 3,5 |
| | et D-Glucose | 10 µg |

6.2 Fonctionnement du dispositif

Le papier D122 est traité à l'aide de la solution suivante :

- Lait écrémé 10 % w/v
- Gélatine 1 %
- Exc Eau distillé stérile.

La goutte de sang total déposée sur l'empilement des pastilles reste immobile 30 secondes grâce à la 1ère MLP.

Cette derniere est lysée au bout de 30 secondes par le détergent contenu dans la solution de dépose des antigènes marqués (le Tween 20 0,1 %) qui a d'ailleurs trois autres fonctions très importantes :

- protéger la péroxydase des antigènes,
- accélérer l'hemolyse,
- servir d'agent de blocking : évite aux antigènes marqués de se fixer au D122 en cas de long stockage.

Les anticorps sériques éventuels, se lient aux antigènes marqués contenus dans la solution de dépot du papier D122 :

- Tween 20 0,1 %
- Gélatine 1 %
- Antigènes marqués Glucose Oxydase 10 ng/µl
- Exc PBS

Dépôt de 5 µl de cette solution.

En cas de négatif, les antigènes marqués disposent de 30 secondes (temps de lyse de la 2ème MLP) pour se fixer sur les anticorps de la phase solide.

En cas de positif, le complexe anticorps sériques-antigènes marqués ne se fixe pas sur la phase solide et la glucose oxydase marquant les antigènes produit de l'eau oxygénée au contact du D-Glucose de la pastille de révélation procurant ainsi son substrat ($H_2O_2$) à la péroxydase de l'hémoglobine pour colorer le TMB.

Après 30 secondes la 2ème MLP est lysée et le complexe anticorps recherchés + les antigènes marqués à la glucose-oxydase arrivent sur la pastille de révélation.

Exemple 7. Test semi qualitatif de dosage d'ACE dans du sang total.

Comme indiqué plus haut, en relation avec la figure 1, dans les tests semi-qualitatifs conformes à la présente invention, le dispositif comprend une première phase solide qui piége une quantité prédéterminée d'antigènes correspondant à une valeur normale, tandis que les antigènes présents dans le sang au-delà de cette valeur dite normale, sont piégés par une seconde phase solide.

Le dispositif mis en oeuvre dans ce test est le suivant :

Description du dispositif :

| | |
|---|---|
| Phase solide<br>Nylon traité | Anticorps antiACE fixé piégeant 5 nanogrammes d'ACE/ml de sang |
| 1ère MLP en lipides | |
| Papier D122 traité | Anticorps antiACE marqués 50 ng à la Gluc. Oxydase se lient à l'ACE exced. |
| 2ème MLP lipides | |
| Phase solide n° 2 | Antigènes fixés piégent les anticorps marqués non saturés par ACE |
| 3ème MLP lipides | |
| Support Nylon | TMB 3 µl<br>Tampon Citrate 1M 10 µl<br>D-Glucose 10 µg |

Exemple 8 . Test qualitatif de détection de HCG dans les urines (test qualitatif de grossesse)

Le dispositif conforme à l'invention présente la stucture suivante :

| Matériau utilisé | Substance contenue | Quantité |
|---|---|---|
| 1 - Papier Durieux 122 | Tampon Phosphate 1M | 10 µl |
| 2 - Papier traité | Anticorps marqués | 50 ng |
| 3 - Membrane à lyse programmée | Copolymère d'acrylate | |
| 4 - Microbilles en gel sur Nylon | Antigène recherché | excès puis lavages et blocking |
| 5 - Membrane à lyse programmée | Copolymère d'acrylate | |
| 6 - Papier D 122 | D-Glucose sec | 50 µg |
| 7 - Papier D 122 | Glucose-oxydase | 2 U.I. |
| 8 - Nylon Pores 0,65 µ traité au | 3-3'-5-5' TMB<br>Tampon citrate pH 3,5 | 2,5 µg<br>10 µl 1M |

Les huit couches qui viennent d'être décrites sont superposées et pressées avec une lamelle de plexiglas propre, perforée d'un orifice de 2 à 8 mm de diamètre.

A l'aide d'une pipette Gilson on prélève 90 µl d'urines fraichement émises et on les dépose doucement dans l'orifice.

Une faible partie des urines est immédiatement absorbée et la goutte déposée reste immobile pendant 20 secondes puis s'affaisse brusquement et partiellement : les pastilles supérieures restent humides. Après 20 secondes, nouvel affaissement du niveau des urines et le dispositif vu d'en haut apparait complètement sec.

Ces deux étapes illustrent bien la lyse programmée des deux membranes situées l'une sous les anticorps marqués et l'autre sous la phase solide.

En cas de résultat négatif, l'eau oxygénée produite, donne en l'absence des anticorps-péroxydase, une coloration saumonée au bout de 1-2 minutes. (résultat de la lumière et $H_2O_2$ sur le TMB)

En cas de réaction positive, un spot bleu azur intense apparait immédiatement.

Pour conserver les couleurs, il est impératif de les garder au sec et surtout à l'obscurité. En pratique, il faudra simplement remettre le test dans son emballage et bien fermer si on désire montrer le résultat plus tard. Dans ces conditions, la coloration dure plusieurs jours.

Exemple 9. Test semi-quantitatif de détection du pic de LH dans les urines

La LH urinaire est présente à un niveau basal pendant tout le cycle. Elle connait un pic important peu avant et pendant l'ovulation. L'objectif du test est donc de ne pas provoquer de coloration en cas de niveau basal de LH et de donner un signal coloré en cas de pic de LH.

Pour cela, il est ajouté un étage à la partie supérieure, au dessus des anticorps marqués. Cet étage comprend :

- une phase solide préparée avec des anticorps anti-LH de telle sorte que seule la quantité de LH correspondant au niveau basal soit piégée à ce niveau,
- une membrane à lyse programmée laissant le temps aux anticorps anti-LH de cette phase solide de piéger le niveau basal de LH.

Le reste du dispositif est identique au qualitatif.

| | | |
|---|---|---|
| Dispositif : | | |
| Papier Durieux 122 | Tampon phosphate 1M | 10 µl |
| Phase solide N° 1 : gel de microbilles | Anticorps Anti-LH | Niveau basal |
| Membrane à lyse programmée N° 1 : acrylate copolymère (en abrégé : MLP) | | |
| Papier D122 préparé | Anticorps Anti-LH marqués à la péroxydase | 50 ng |
| MLP N° 2 | | |
| Phase solide N° 2 : gel de microbilles | LH (Antigène) | excès puis lavage et blocking |
| MLP N° 3 | | |
| D.122 | D-Glucose et Glucose Oxydase | |
| Nylon | TMB | Spot de 2-3 µl |
| | + Tampon Citrate 1M | 10 µl |

En variante, les anticorps marqués anti-LH peuvent être emprisonnés dans des liposomes au niveau de la PS N° 1 pour laisser en priorité les anticorps anti-LH de la PS N° 1 piéger le niveau basal de LH.

## Revendications

1. Dispositif pour la détermination qualitative et quantitative rapide de la présence d'un ligand réactif, dans un fluide, du type comprenant une zone de réaction contenant un réactif marqué, notamment un anticorps ou un antigène, marqué, destiné à réagir avec le ligand recherché, et une zone de révélation, lequel dispositif est caractérisé en ce qu'il comprend, en combinaison :
   - une première zone de réaction (13) qui comprend une membrane (3), au moins temporairement imperméable, laquelle zone est prévue pour recevoir un échantillon de fluide à tester et laquelle membrane est destinée à être associée à au moins un réactif convenablement marqué, apte à reconnaître le ou les ligands recherché(s) éventuellement contenu(s) dans ledit échantillon, et à une substance de dissolution ou un agent de lyse de ladite membrane ;
   - une deuxième zone de réaction (11), distincte de la première et au moins temporairement isolée de celleci par la membrane (3) susdite, comprenant une phase solide contenant un réactif de référence, non marqué (5 ou 20), apte à reconnaître le ou les ligand(s) que l'on cherche à identifier et au moins temporairement close du côté opposé à celui où se trouve la première membrane, par une deuxième membrane (3') au moins temporairement imperméable, et
   - une troisième zone de réaction (16) au moins temporairement isolée de la deuxième zone de réaction susdite par ladite deuxième membrane (3'), qui contient des moyens de révélation de la réaction qui s'est éventuellement produite dans la première ou la deuxième zone de réaction, ledit dispositif étant en outre caractérisé en ce que les deux membranes (3, 3') susdites sont réalisées en un matériau imperméable pendant une première période de temps suffisante pour permettre à la réaction qui doit se produire dans la zone de réaction intéressée, d'avoir lieu, ledit matériau étant tel qu'il est dissous ou lysé à l'aide de la substance de dissolution ou l'agent de lyse, au bout de cette première période de temps, pour laisser passer d'éventuels produits de la réaction vers la zone de réaction suivante, laquelle dissolution ou lyse débute simultanément à la réaction qui doit se produire dans la zone de réaction intéressée.

2. Dispositif selon la Revendication 1, caractérisé en ce que la substance de dissolution ou de lyse des membrane est indépendante de la membrane concernée.

3. Dispositif selon l'une quelconque des Revendications 1, caractérisé en ce que la substance de dissolution

ou de lyse des membrane(s) est incorporée à la membrane concernée.

4. Dispositif selon l'une quelconque des Revendications 1 à 3, caractérisé en ce que la première zone de réaction (13) est propre à recevoir une phase solide (1), avantageusement supportée par la première membrane (3), qui comprend au moins un réactif convenablement marqué, éventuellement associé à un agent de lyse de ladite membrane.

5. Dispositif selon l'une quelconque des Revendications 1 à 4, caractérisé en ce que la phase solide contenant un réactif de référence, non marqué, qui se trouve dans la deuxième zone de réaction (11), est constituée par un support insoluble (5 ou 20) sur lequel est fixé ledit réactif (anticorps ou antigène, notamment) , non marqué, qui correspond au ligand que l'on cherche à détecter.

6. Dispositif selon l'une quelconque des Revendications 1 à 5, caractérisé en ce que la troisième zone de réaction (16) contient un substrat chromogène lorsqu'il est prévu que la lecture de la réaction doit se faire par colorimétrie.

7. Dispositif selon l'une quelconque des Revendications 1 à 6, caractérisé en ce que le dispositif comporte entre la première et la deuxième zones de réaction, une zone intermédiaire qui comprend un réactif marqué, auquel cas la première zone de réaction comprend un réactif non marqué.

8. Dispositif selon la Revendication 7, caractérisé en ce que la zone intermédiaire comprend au moins une couche de structures vésiculaires multicouches dans lesquelles est incorporé au moins un réactif propre à être libéré au bout d'une temps approprié par un agent de lyse approprié, tel, notamment, qu'une enzyme.

9. Dispositif selon la Revendication 8, caractérisé en ce que la zone intermédiaire est formée d'une membrane intermédiaire intercalée entre la première membrane susdite et la deuxième zone de réaction, laquelle membrane intermédiaire est avantageusement associée à un réactif marqué, auquel cas la première membrane est associée à un réactif de même nature que la membrane intermédiaire susdite.

10. Dispositif selon l'une quelconque des Revendications 1 à 9, caractérisé en ce que la troisième zone dudit dispositif est associée à un appareillage de quantification connu en lui-même, tel que RIA, spectrophotomètre, entre autres.

11. Dispositif selon l'une quelconque des Revendications 1 à 10, caractérisé en ce que les zones de réaction susdites sont superposées pour former un étage supérieur contenant la première zone de réaction, un étage médian contenant la deuxième zone de réaction et un étage inférieur contenant la troisième zone de réaction, et éventuellement un étage intermédiaire entre l'étage supérieur et l'étage médian, qui contient la zone de réaction intermédiaire.

12. Procédé de détermination qualitative et quantitative rapide de la présence d'un ligand réactif dans un fluide, caractérisé en ce qu'au cours d'une première étape, un échantillon du fluide à analyser est mis en contact avec un réactif marqué, notamment tel qu'un anticorps ou un antigène marqué, dans une zone temporairement isolée des zones dans lesquelles ont lieu les étapes ultérieures du procédé, pendant un temps suffisant pour permettre à la réaction ligand-réactif de se produire, temps au bout duquel l'isolement de la zone dans laquelle a eu lieu ladite réaction, est rompu à l'aide d'une substance de dissolution ou d'un agent de lyse approprié présent dans ladite zone, transporté simultanément audit fluide vers une membrane temporairement imperméable qui est dissoute au contact de ladite substance ou agent, -laquelle dissolution ou lyse progressive et programmée de la membrane d'isolement est simultanée à ladite réaction-, pour permettre au produit éventuel de la réaction d'entrer en contact, au cours d'une deuxième étape, avec un second réactif, non marqué, correspondant au ligand que l'on cherche à identifier dans l'échantillon de fluide susdit, dans une seconde zone temporairement isolée de la zone qui lui fait suite, après quoi l'isolement de la seconde zone est rompu de manière progressive, programmée et simultanée à l'aide de ladite substance de dissolution ou dudit agent de lyse pour permettre au produit de la première ou de la deuxième réaction d'entrer en contact au cours de la troisième étape du procédé, avec des moyens de révélation de la présence - ou de l'absence - du ligand que l'on cherche à détecter.

13. Procédé selon la Revendication 12, caractérisé en ce que l'échantillon de fluide est mis en contact, au cours de la première étape du procédé, avec un réactif non marqué et la seconde étape du procédé est

précédée d'une étape intermédiaire au cours de laquelle le complexe ligand-réactif non marqué formé au cours de la première étape, est mis en contact avec un réactif marqué de même nature que le réactif mis en oeuvre dans la première étape, dans une zone temporairement isolée de l'étape suivante.

**14.** Procédé selon la Revendication 13, caractérisé en ce que le réactif non marqué mis en contact avec l'échantillon de fluide à analyser, au cours de la première étape du procédé, est introduit à raison d'une quantité déterminée qui correspond à une quantité connue de ligand tel que celui que l'on cherche à détecter, le ligand en excès, c'est-à-dire non lié au réactif au cours de la première étape, est piégé, au cours de l'étape intermédiaire du procédé, par le réactif marqué sur lequel ledit excès de ligand se fixe, d'où il résulte que la réaction de révélation de la présence éventuelle du ligand dans le fluide à analyser n'est positive que si le taux de ligand dans ledit fluide est supérieur à la quantité susdite, permettant ainsi une détermination semi-quantitative du ligand présent dans ledit fluide.

**15.** Procédé selon l'une quelconque des Revendications 12 à 14, caractérisé en ce que la première étape du procédé est une phase d'absorption et la deuxième étape du procédé est une phase de désorption, tandis que la troisième étape du procédé est une phase d'absorption.

**16.** Procédé selon la Revendication 15, caractérisé en ce que lorsqu'il comporte une étape intermédiaire intercalée entre la première et la deuxième étapes du procédé, cette étape intermédiaire est, comme la première étape, une phase d'absorption.

**Patentansprüche**

**1.** Vorrichtung zur raschen qualitativen und quantitativen Bestimmung des Vorhandenseins eines reaktiven Liganden in einer Flüssigkeit, des Typs umfassend eine Reaktionszone, die ein markiertes Reagens, insbesondere einen Antikörper oder ein Antigen, der bzw. das so markiert ist, daß es mit dem gewünschten Liganden reagieren kann, enthält und eine Nachweiszone, dadurch **gekennzeichnet**, daß sie in Kombination umfaßt:

- eine erste Reaktionszone (13), die eine Membran (3) umfaßt, die mindestens zeitweise undurchlässig ist, wobei die Zone für die Aufnahme einer zu testenden Probenflüssigkeit vorgesehen ist, und wobei die Membran zur Assoziation mit mindestens einem in geeigneter Weise markierten Reagens, das den bzw. die gesuchten Liganden, der bzw. die gegebenenfalls in der Probe enthalten ist bzw. sind, erkennen kann, und mit einer Substanz zur Auflösung oder einem Mittel zur Lyse der Membran vorgesehen ist;
- eine zweite Reaktionszone (11), die sich von der ersten unterscheidet und mindestens zeitweise von dieser durch die Membran (3) isoliert ist, umfassend eine feste Phase, die ein nicht-markiertes Referenzreagens (5 oder 20) enthält, das den bzw. die Liganden, die identifiziert werden sollen, erkennen kann und die mindestens zeitweise gegen die Seite, die derjenigen, in der sich die erste Membran befindet, durch eine zweite Membran (3'), die mindestens zeitweise undurchlässig ist, abgeschlossen ist, und
- eine dritte Reaktionszone (16), die mindestens zeitweise gegen die zweite Reaktionszone durch die zweite Membran (3') isoliert ist, die Mittel zum Nachweis der Reaktion, die gegebenenfalls in der ersten oder der zweiten Reaktionszone abläuft, enthält, wobei die Vorrichtung weiterhin dadurch **gekennzeichnet** ist, daß die zwei Membranen (3, 3') aus einem Material hergestellt sind, das während einer ersten Zeitspanne, die ausreicht, die Reaktion, die in der Reaktionszone von Interesse abläuft, ablaufen zu lassen, undurchlässig ist, wobei das Material so ist, daß es mit Hilfe der Substanz zur Auflösung oder dem Lysemittel im Verlauf dieser ersten Zeitspanne aufgelöst wird, um evtl. vorhandene Reaktionsprodukte gegen die nachfolgende Reaktionszone fließen zu lassen, wobei die Auflösung oder Lyse gleichzeitig mit der Reaktion, die in der Reaktionszone von Interesse ablaufen soll, beginnt.

**2.** Vorrichtung nach Anspruch 1, dadurch **gekennzeichnet**, daß die Substanz zur Auflösung oder Lyse der Membran unabhängig von der betroffenen Membran ist.

**3.** Vorrichtung nach einem der Ansprüche 1 oder 2, dadurch **gekennzeichnet**, daß die Substanz zur Auflösung oder zur Lyse der Membran(en) in die betroffene Membran eingearbeitet ist.

**4.** Vorrichtung nach einem der Ansprüche 1 bis 3, dadurch **gekennzeichnet**, daß die erste Reaktionszone

(13) für die Aufnahme einer festen Phase (1), die bevorzugt auf der ersten Membran (3) aufgebracht ist, geeignet ist, die mindestens ein in geeigneter Weise markiertes Reagens enthält, das gegebenenfalls mit einem Mittel zur Lyse der Membran assoziiert ist.

5. Vorrichtung nach einem der Ansprüche 1 bis 4, dadurch **gekennzeichnet**, daß sich die feste Phase, die ein nicht-markiertes Referenzreagens enthält, in der zweiten Reaktionszone (11) befindet und aus einem unlöslichen Träger (5 oder 20) besteht, auf den das Reagens (insbesondere ein Antikörper oder ein Antigen), das nicht markiert ist, fixiert ist, das dem nachzuweisenden Liganden entspricht.

6. Vorrichtung nach einem der Ansprüche 1 bis 5, dadurch **gekennzeichnet**, daß die dritte Reaktionszone (16) ein chromogenes Substrat enthält, wenn das Ablesen der Reaktion colorimetrisch vorgenommen werden soll.

7. Vorrichtung nach einem der Ansprüche 1 bis 6, dadurch **gekennzeichnet**, daß die Vorrichtung zwischen den ersten und zweiten Reaktionszonen eine intermediäre Zone enthält, die ein markiertes Reagens enthält, wobei in diesem Falle die erste Reaktionszone ein nicht-markiertes Reagens enthält.

8. Vorrichtung nach Anspruch 7, dadurch **gekennzeichnet**, daß die intermediäre Zone mindestens eine Schicht aus mehrschichtigen vesikulären Strukturen umfaßt, in die mindestens ein Reagens, das im Verlauf einer geeigneten Zeit durch ein geeignetes Lysemittel, insbesondere ein Enzym, freigesetzt werden kann, eingearbeitet ist.

9. Vorrichtung nach Anspruch 8, dadurch **gekennzeichnet**, daß die intermediäre Zone aus einer intermediären Membran gebildet ist, die zwischen der ersten Membran und der zweiten Reaktionszone angebracht ist, wobei an die intermediäre Membran bevorzugt markiertes Reagens gebunden ist, und in diesem Falle an die erste Membran ein Reagens der gleichen Art wie die intermediäre Membran gebunden ist.

10. Vorrichtung nach einem der Ansprüche 1 bis 9, dadurch **gekennzeichnet**, daß die dritte Zone der Vorrichtung mit einer an sich bekannten Vorrichtung zur quantitativen Bestimmung, wie u.a. einem RIA, einem Spektrophotometer, verbunden ist.

11. Vorrichtung nach einem der Ansprüche 1 bis 10, dadurch **gekennzeichnet**, daß die Reaktionszonen übereinander angeordnet sind, um eine obere Schicht, die die erste Reaktionszone enthält, eine mittlere Schicht, die die zweite Reaktionszone enthält und eine untere Schicht, die die dritte Reaktionszone enthält und gegebenenfalls eine Zwischenschicht zwischen der oberen und der mittleren Schicht, die die intermediäre Reaktionzone enthält, zu bilden.

12. Verfahren zur raschen qualitativen und quantitativen Bestimmung des Vorhandenseins eines reaktiven Liganden in einer Flüssigkeit, dadurch **gekennzeichnet**, daß im Verlauf einer ersten Stufe eine zu analysierende Probenflüssigkeit in Kontakt mit einem markierten Reagens, insbesondere einem markierten Antikörper oder einem markierten Antigen in einer zeitweise gegen die Zonen, in denen die nachfolgenden Verfahrensstufen stattfinden, isoliert ist, eine ausreichende Zeit lang in Kontakt gebracht wird, um den Ablauf der Reaktion zwischen dem Liganden und dem Reagens zu gestatten, wobei im Verlauf dieser Zeit die Isolierung der Zone, in der die Reaktion stattgefunden hat, mit Hilfe einer geeigneten Substanz zur Auflösung oder eines geeigneten Lysemittels, die bzw. das in der Zone vorhanden ist, die bzw. das gleichzeitig mit der Flüssigkeit gegen eine zeitweise undurchlässige Membran, die bei Kontakt mit der Substanz oder dem Reagens aufgelöst wird, transportiert wird, unterbrochen wird - die schrittweise und programmierte Auflösung oder Lyse der Isolierungsmembran findet gleichzeitig mit der Reaktion statt - um zu gestatten, daß das gegebenenfalls vorhandene Reaktionsprodukt im Verlauf einer zweiten Stufe mit einem zweiten nicht-markierten Reagens, das den Liganden, der in der Flüssigkeitsprobe nachgewiesen werden soll entspricht, in einer zweiten Zone, die zeitweise gegen die Zone, die darauf folgt, isoliert ist, in Kontakt tritt, wonach die Isolierung der zweiten Zone schrittweise programmiert und gleichzeitig mit Hilfe der Substanz zur Auflösung oder des Lysemittels unterbrochen wird, um zu gestatten, daß das Produkt der ersten oder der zweiten Reaktion im Verlauf der dritten Verfahrensstufe mit Mitteln zum Nachweis des Vorhandenseins oder des Fehlens des nachzuweisenden Liganden in Kontakt tritt.

13. Verfahren nach Anspruch 12, dadurch **gekennzeichnet**, daß die Flüssigkeitsprobe im Verlauf der ersten Verfahrensstufe mit einem nicht-markierten Reagens in Kontakt gebracht wird, und daß der zweiten Verfahrensstufe eine intermediäre Stufe vorausgeht, im Verlauf derer der im Verlauf der ersten Stufe gebildete Komplex aus Ligand und nicht-markiertem Reagens mit einem markierten Reagens der gleichen Art wie das in der ersten Stufe verwendete Reagens in einer zeitweise gegen die nachfolgende Stufe isolierten Zone in Kontakt gebracht wird.

**14.** Verfahren nach Anspruch 13, dadurch **gekennzeichnet**, daß das nicht-markierte Reagens, das mit der zu analysierenden Flüssigkeitsprobe in Kontakt gebracht wird, im Verlauf der ersten Verfahrensstufe, bezogen auf eine bestimmte Menge, die einer bekannten Menge des Liganden, wie dem, der nachgewiesen werden soll, entspricht, eingebracht wird, wobei der Ligand, der im Überschuß vorhanden ist, d.h. der an das Reagens im Verlauf der ersten Stufe nicht gebunden wird, im Verlauf der intermediären Verfahrensstufe durch das markierte Reagens eingefangen wird, auf dem ein Überschuß des Liganden gebunden wird, woraus folgt, daß die Reaktion zum Nachweis des eventuellen Vorhandenseins des Liganden in der Analysenflüssigkeit nicht positiv ist, wenn der Gehalt des Liganden in der Flüssigkeit größer als diese Menge ist, was so eine semi-quantitative Bestimmung des in der Flüssigkeit vorhandenen Liganden gestattet.

**15.** Verfahren nach einem der Ansprüche 12 bis 14, dadurch **gekennzeichnet**, daß die erste Verfahrensstufe eine Phase der Absorption ist, und daß die zweite Verfahrensstufe eine Phase der Desorption ist, während die dritte Verfahrensstufe eine Phase der Absorption ist.

**16.** Verfahren nach Anspurcn 15, dadurch **gekennzeichnet**, daß wenn eine intermediäre Stufe zwischen den ersten und zweiten Verfahrensstufen vorhanden ist, diese intermediäre Stufe, wie die erste Stufe, eine Absorptionsphase ist.

## Claims

**1.** Device for the rapid qualitative and quantitative determination of the presence of a reactive ligand, in a fluid, of the type comprising a reaction zone containing a labelled reagent, especially a labelled antibody or antigen, intended to react with the desired ligand, and a visualising zone, which device is characterised in that it comprises, in combination:

- a first reaction zone (13) which comprises a membrane (3) which is at least temporarily impermeable, which zone is intended to receive a sample of test fluid and which membrane is intended to be combined with at least one suitably labelled reagent capable of recognising the desired ligand(s) which may be contained in the said sample, and with a substance for dissolving or an agent for lysing the said membrane:
- a second reaction zone (11), distinct from the first and at least temporarily isolated from the latter by the abovementioned membrane (3), comprising a solid phase containing an unlabelled reference reagent (5 or 20) capable of recognising the ligand(s) which it is desired to identify and at least temporarily closed on the side opposite that on which the first membrane is present, by a second membrane (3′) which is at least temporarily impermeable, and
- a third reaction zone (16), at least temporarily isolated from the abovementioned second reaction zone by the said second membrane (3′), which contains means for visualising the reaction which may have occurred in the first or second reaction zone, the said device being additionally characterised in that the two abovementioned membranes (3, 3′) are produced from a material which is impermeable for a first period of time sufficient to allow the reaction which should occur in the relevant reaction zone to take place, the said material being such that it is dissolved or lysed by means of the dissolving substance or lysing agent at the end of this first period of time, in order to allow possible reaction products to pass to the next reaction zone, which dissolution or lysis starts simultaneously with the reaction which should occur in the relevant reaction zone.

**2.** Device according to Claim 1, characterised in that the substance for dissolving or lysing the membranes is independent of the relevant membrane.

**3.** Device according to any one of Claims 1 to 2, characterised in that the substance for dissolving or lysing the membrane(s) is incorporated into the relevant membrane.

**4.** Device according to any one of Claims 1 to 3, characterised in that the first reaction zone (13) is suitable for receiving a solid phase (1), advantageously supported by the first membrane (3), which comprises at least one suitably labelled reagent, optionally combined with an agent for lysing the said membrane.

5. Device according to any one of Claims 1 to 4, characterised in that the solid phase containing an unlabelled reference reagent, which is present in the second reaction zone (11), consists of an insoluble support (5 or 20) onto which the said unlabelled reagent (especially antibody or antigen), which corresponds to the ligand which it is desired to detect, is bound.

6. Device according to any one of Claims 1 to 5, characterised in that the third reaction zone (16) contains a chromogenic substrate when it is intended that the reaction should be read by colorimetry.

7. Device according to any one of Claims 1 to 6, characterised in that the device comprises, between the first and second reaction zones, an intermediate zone which comprises a labelled reagent, in which case the first reaction zone comprises an unlabelled reagent.

8. Device according to Claim 7, characterised in that the intermediate zone comprises at least one layer of multilayer vesicular structures in which at least one reagent capable of being released at the end of an appropriate time by an appropriate lysing agent such as, especially, an enzyme, is incorporated.

9. Device according to Claim 8, characterised in that the intermediate zone consists of an intermediate membrane interpolated between the abovementioned first membrane and the second reaction zone, which intermediate membrane is advantageously combined with a labelled reagent, in which case the first membrane is combined with a reagent of the same nature as the abovementioned intermediate membrane.

10. Device according to any one of Claims 1 to 9, characterised in that the third zone of the said device is combined with a quantifying apparatus known per se, such as RIA, spectrophotometer, among others.

11. Device according to any one of Claims 1 to 10, characterised in that the abovementioned reaction zones are superposed to form an upper level containing the first reaction zone, a middle level containing the second reaction zone and a lower level containing the third reaction zone, and optionally an intermediate level between the upper level and the middle level, which contains the intermediate reaction zone.

12. Process for the rapid qualitative and quantitative determination of the presence of a reactive ligand in a fluid, characterised in that during a first stage, a sample of fluid to be analysed is placed in contact with a labelled reagent, especially such as a labelled antibody or antigen, in a zone temporarily isolated from the zones in which the subsequent steps of the process take place, for a time sufficient to allow the ligand-reagent reaction to occur, at the end of which time the isolation of the zone in which the said reaction took place is broken by means of an appropriate dissolving substance or lysing agent present in the said zone, transported simultaneously with the said fluid to a temporarily impermeable membrane which is dissolved in contact with the said substance or agent - which gradual and programmed dissolution or lysis of the isolating membrane is simultaneous with the said reaction - in order to allow the possible reaction product to come into contact, during a second stage, with a second unlabelled reagent corresponding to the ligand which it is desired to identify in the abovementioned sample of fluid, in a second zone temporarily isolated from the zone next to it, after which the isolation of the second zone is broken in a gradual, programmed and simultaneous manner by means of the said dissolution substance or the said lysing agent in order to allow the product of the first or second reaction to come into contact, during the third stage of the process, with means for visualising the presence - or the absence - of the ligand which it is desired to detect.

13. Process according to Claim 12, characterised in that the sample of fluid is placed in contact, during the first stage of the process, with an unlabelled reagent and the second stage of the process is preceded by an intermediate stage during which the ligand-unlabelled reagent complex formed during the first stage is placed in contact with a labelled reagent of the same nature as the reagent used in the first stage, in a zone temporarily isolated from the next stage.

14. Process according to Claim 13, characterised in that the unlabelled reagent placed in contact with the sample of fluid to be analysed, during the first stage of the process, is introduced in a determined quantity which corresponds to a known quantity of ligand such as that which it is desired to detect, the excess ligand, that is to say not bound to the reagent during the first stage, is trapped, during the intermediate stage of the process, by the labelled reagent to which the said excess ligand binds, with the result that the reaction for visualising the possible presence of the ligand in the fluid to be analysed is positive only if the ligand level in the said fluid is higher than the abovementioned quantity, thus allowing a semiquantitative

determination of the ligand present in the said fluid.

**15.** Process according to any one of Claims 12 to 14, characterised in that the first stage of the process is an absorption phase and the second stage of the process is a desorption phase, whereas the third stage of the process is an absorption phase.

**16.** Process according to Claim 15, characterised in that when it comprises an intermediate stage intercalated between the first and the second stages of the process, this intermediate stage is, like the first stage, an absorption phase.

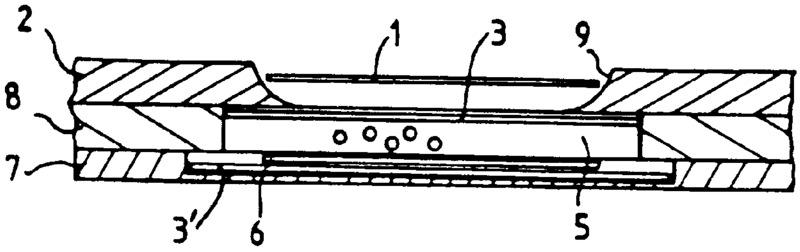
2
1
3
9
8
7
3'
6
5

FIG.1

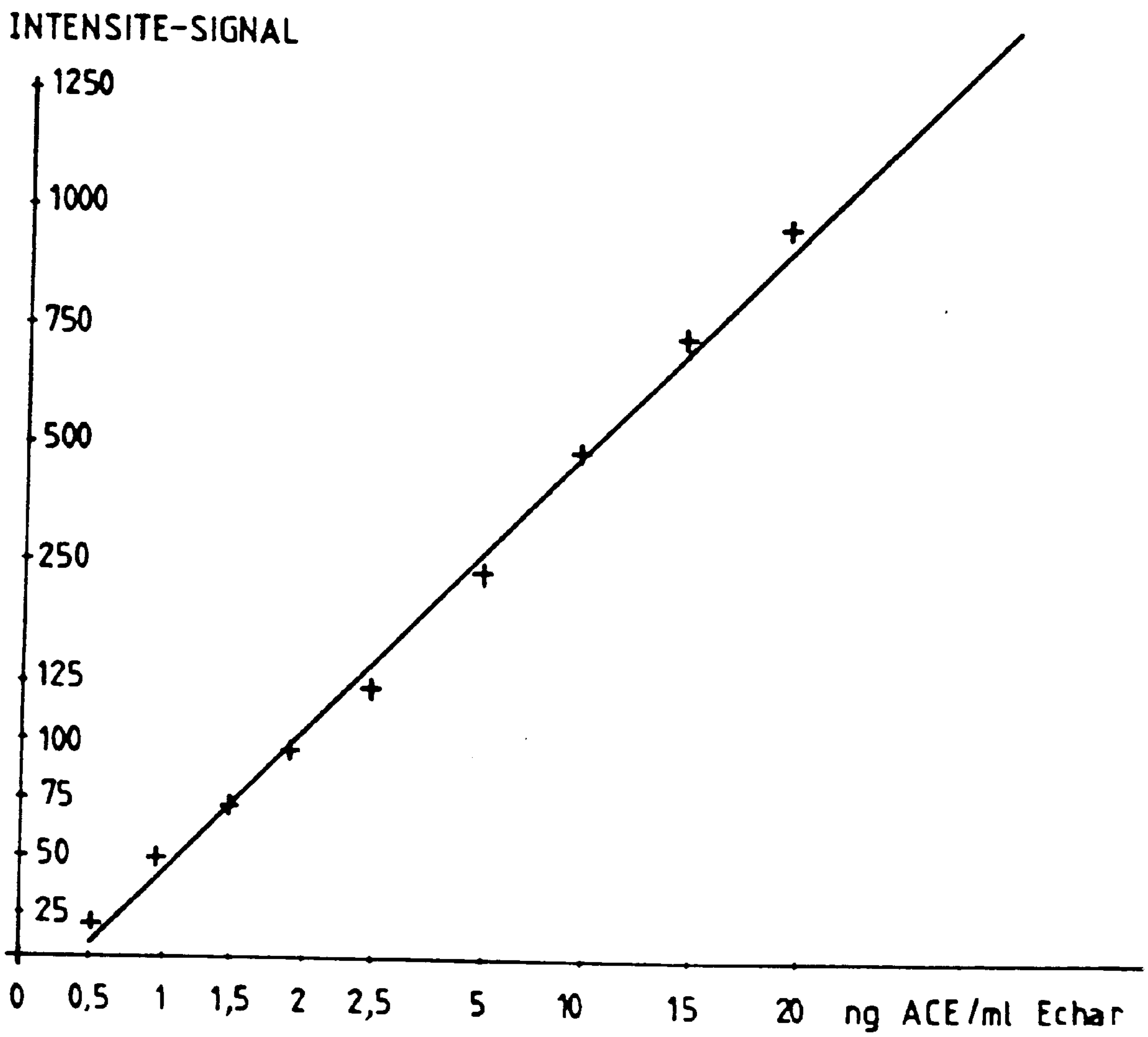
INTENSITE-SIGNAL
1250
1000
750
500
250
125
100
75
50
25
0
0,5
1
1,5
2
2,5
5
10
15
20
ng ACE/ml Echar

FIG. 4

FIG. 2

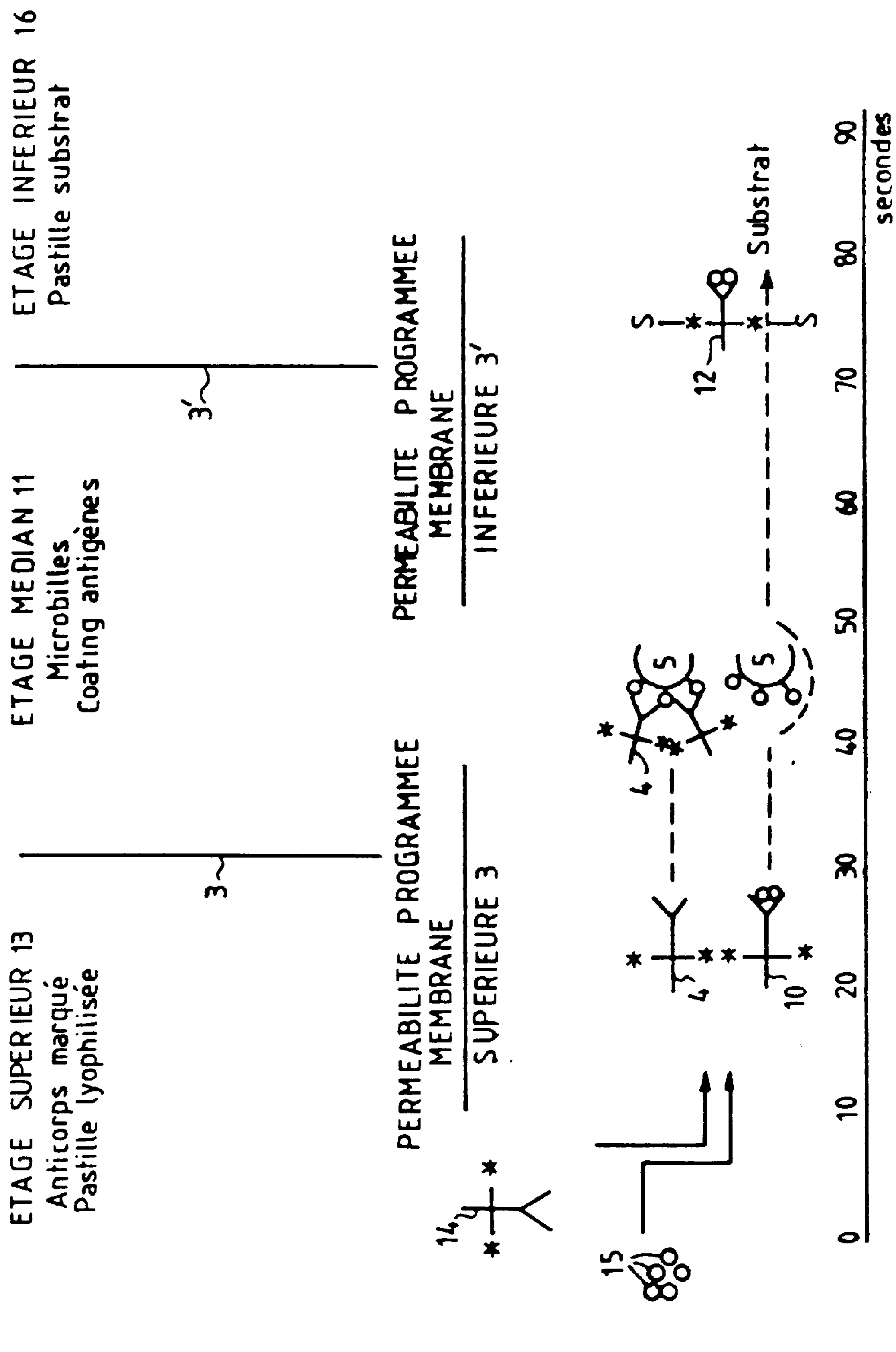
ETAGE SUPERIEUR 13
Anticorps marqué
Pastille lyophilisée
ETAGE MEDIAN 11
Microbilles
Coating antigènes
ETAGE INFERIEUR 16
Pastille substrat
PERMEABILITE PROGRAMMEE MEMBRANE SUPERIEURE 3
PERMEABILITE PROGRAMMEE MEMBRANE INFERIEURE 3'
3
3'
14
15
4
10
S
12
Substrat
0
10
20
30
40
50
60
70
80
90
secondes

FIG.3

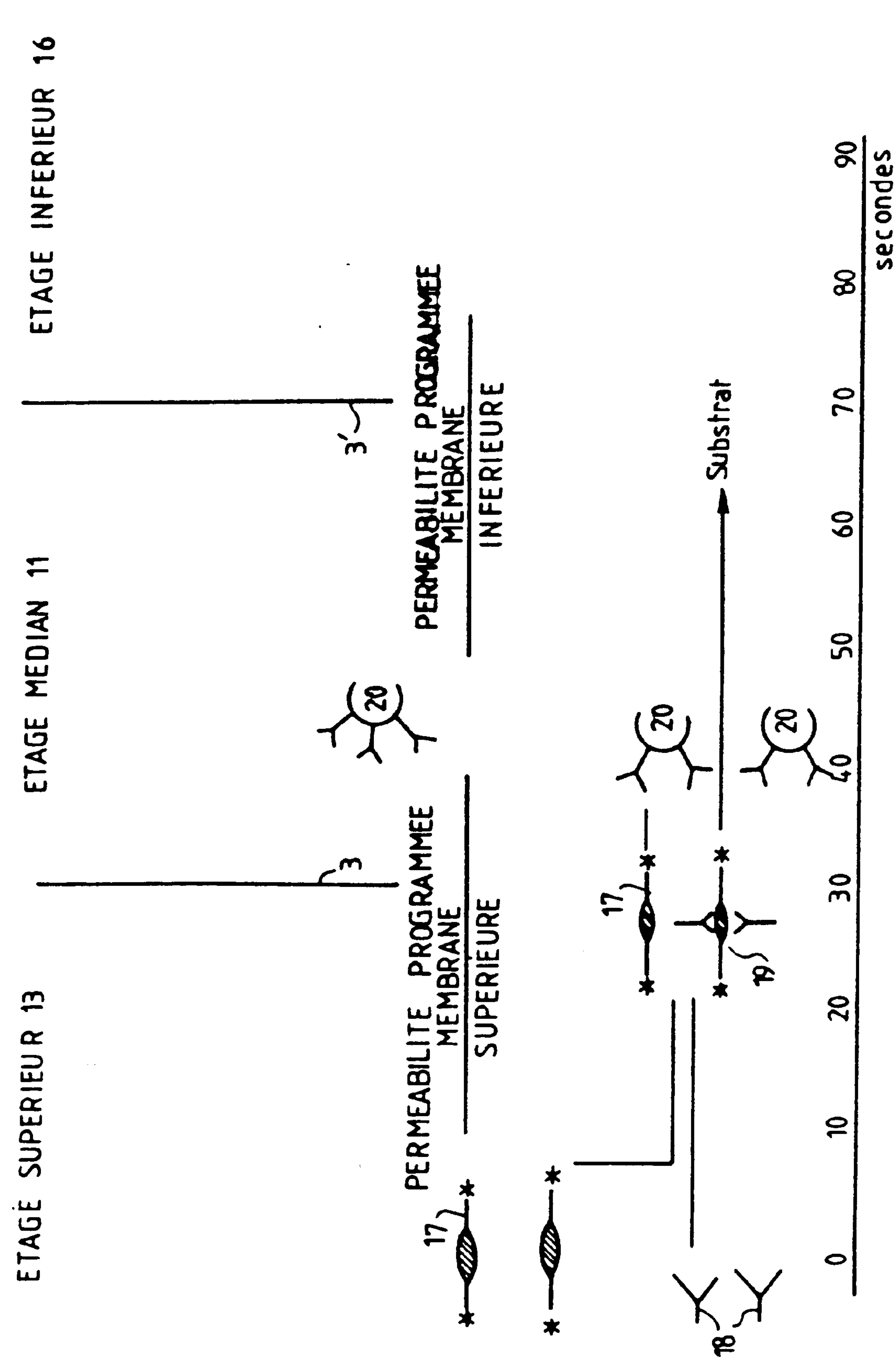
ETAGE SUPERIEUR 13
ETAGE MEDIAN 11
ETAGE INFERIEUR 16
PERMEABILITE PROGRAMMEE MEMBRANE SUPERIEURE
PERMEABILITE PROGRAMMEE MEMBRANE INFERIEURE
3
3'
17
18
19
20
Substrat
0
10
20
30
40
50
60
70
80
90
secondes